# FASCICULE DE BREVET EUROPEEN

(11) **EP 2 404 600 B1**
(45) Date de publication et mention de la délivrance du brevet: **27.05.2015**
(21) Numéro de dépôt: 11290269.7
(22) Date de dépôt: 14.06.2011
(51) Int. Cl.: A61K 31/4045, A61K 31/55, A61K 45/06, A61P 9/04

(54) **Utilisation de l'association d'un inhibiteur du courant If sinusal et d'un inhibiteur de l'enzyme de conversion de l'angiotensine pour le traitement de l'insuffisance cardiaque à fonction systolique conservée**
Verwendung der Verbindung eines IF-Sinusstromhemmers und eines Enzymhemmers zur Umwandlung des Angiotensins für die Behandlung von Herzinsuffizienz mit erhaltener systolischer Funktion
Use of the combination of a sinus current If inhibitor and an inhibitor of the angiotensin conversion enzyme for treating cardiac insufficiency with preserved systolic function

(30) Priorité: 15.06.2010 FR 1002525
(43) Date de publication de la demande: 11.01.2012
(60) Demande divisionnaire: 15162899.7
(73) Titulaire: Les Laboratoires Servier, 92284 Suresnes Cedex (FR)
(72) Inventeur: Thuillez, Christian, 76000 Rouen (FR); Mulder, Paulus, 76130 Mont Saint Aignan (FR); Vilaine, Jean-Paul, 92290 Chatenay Malabry (FR); Fratacci, Marie-Dominique, 78390 Bois d'Arcy (FR); Lerebours-Piegeonniere, Guy, 92300 Levallois-Perret (FR); Feldmann, Luc, 75017 Paris (FR); Roussel, Jérôme, 78220 Viroflay (FR)

(56) Documents cités:
- EP-A1- 1 354 873
- EP-A1- 1 362 590
- EP-A1- 2 036 892
- EP-A1- 2 039 682
- WO-A2-01/78699
- RU-C1- 2 364 401

## Description

La présente invention concerne l'utilisation de l'association de :
- l'ivabradine ou 3-{3-[{[(7S)-3,4-diméthoxybicyclo[4.2.0]octa-1,3,5-trién-7-yl] méthyl}(méthyl)amino]propyl}-7,8-diméthoxy-1,3,4,5-tétrahydro-2*H*-3-benzazépin-2-one de formule (I) : 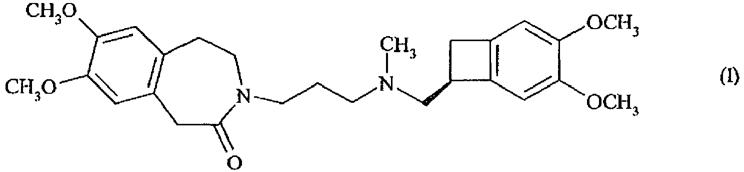 ainsi que ses sels d'addition à un acide pharmaceutiquement acceptable, leurs hydrates et formes cristallines, ou
- la N-{[(7*S*)-3,4-diméthoxybicyclo[4,2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine de formule (II) 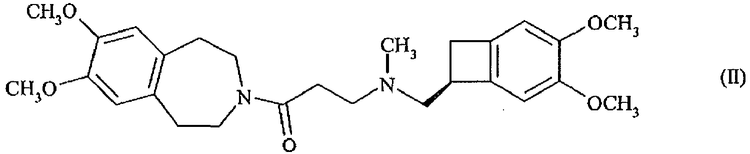 ainsi que ses sels d'addition à un acide pharmaceutiquement acceptable, leurs hydrates et formes cristallines,
avec le perindopril ou un de ses sels d'addition à une base pharmaceutiquement acceptable, leurs hydrates ou formes cristallines,
pour l'obtention de médicaments destinés au traitement de l'insuffisance cardiaque à fonction systolique conservée.

L'ivabradine ainsi que ses sels d'addition à un acide pharmaceutiquement acceptable, et plus particulièrement son chlorhydrate, leurs hydrates et formes cristallines, et la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine, ainsi que ses sels d'addition à un acide pharmaceutiquement acceptable, et plus particulièrement son chlorhydrate et son fumarate, leurs hydrates et formes cristallines, sont des inhibiteurs sélectifs et spécifiques du courant I_{f} sinusal et possèdent des propriétés pharmacologiques et thérapeutiques très intéressantes, notamment des propriétés chronotropes négatives (réduction de la fréquence cardiaque), qui rendent ces composés utiles dans le traitement, la prévention et l'amélioration du pronostic de différentes maladies cardio-vasculaires liées à l'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque chronique.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

La préparation et l'utilisation en thérapeutique de la *N*-{[(7*S*)-3,4-diméthoxybicyclo[4.2.0]octa-1,3,5-trién-7-yl]méthyl}-3-(7,8-diméthoxy-1,2,4,5-tétrahydro -3*H*-3-benzazépin-3-yl)-*N*-méthyl-3-oxo-1-propanamine et de ses sels d'addition à un acide pharmaceutiquement acceptable, et plus particulièrement de son chlorhydrate et de son fumarate, ont été décrits dans le brevet européen EP 2 036 892.

Le perindopril est un inhibiteur de l'enzyme de conversion de l'angiotensine.

Les inhibiteurs de l'enzyme de conversion de l'angiotensine sont une des classes thérapeutiques majeures dans le traitement de l'hypertension artérielle. Ils agissent principalement par l'inhibition de la synthèse de l'angiotensine II et par un blocage de la dégradation de la bradykinine.

Ils ont montré qu'au delà de la baisse de la pression artérielle ils amélioraient la morbidité (infarctus du myocarde, accidents vasculaires cérébraux) et la mortalité cardiovasculaire des hypertendus, des diabétiques, des malades avec une maladie coronaire préexistante. Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique, patnoïque, 1,5-naphtalènedisulfonique.

La demanderesse a découvert que l'association de :
- l'ivabradine ou 3-{3-[{[(7S)-3,4-diméthoxybicyclo[4.2.0]octa-1,3,5-trién-7-yl] méthyl}(méthyl)amino]propyl}-7,8-diméthoxy-1,3,4,5-tétrahydro-2*H*-3-benzazépin-2-one, ou
- la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine,
et du perindopril, possédait des propriétés intéressantes permettant son utilisation dans le traitement de l'insuffisance cardiaque à fonction systolique conservée.

L'insuffisance cardiaque par dysfonction systolique du ventricule gauche n'est plus la seule forme d'insuffisance cardiaque. De plus en plus souvent, les patients qui ont une insuffisance cardiaque ont une fraction d'éjection supérieure à 40 %. La proportion d'insuffisance cardiaque dite « diastolique » (ou plutôt « à fonction systolique conservée ») augmente avec l'âge. Elle rend compte actuellement de 30 à 40 % des hospitalisations pour insuffisance cardiaque et, après 80 ans, dépasse en fréquence celle des insuffisances cardiaques par dysfonction systolique. Les insuffisances cardiaques diastoliques associent généralement une prolongation de la relaxation ventriculaire et une réduction de la distensibilité de la chambre ventriculaire gauche. Les causes essentielles sont les cardiopathies ischémiques, hypertensives et du sujet âgé. Les facteurs prédisposant sont l'age, le sexe (femme), le diabète, l'obésité et l'hypertension artérielle. Le remodelage concentrique du ventricule gauche, avec ou sans hypertrophie, entraîne constamment un trouble de la fonction diastolique. On trouve le plus souvent un facteur déclenchant à l'origine d'une poussée congestive. L'insuffisance cardiaque dite diastolique verra sa fréquence croître avec l'âge. Sa physiopathologie reste complexe et justifie d'être mieux comprise par les cliniciens.

Aucun traitement n'a aujourd'hui démontré d'efficacité dans cette pathologie, dont la mortalité, de 50% à 4 ans, est équivalente à celle de l'insuffisance cardiaque systolique.

La demanderesse a découvert que l'utilisation de l'association de l'ivabradine ou de la *N-*{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine et du perindopril permettait d'obtenir des effets pharmacologiques supérieurs à ceux observés en utilisant soit l'ivabradine ou de la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propananline seules, soit le perindopril seul. De plus, l'utilisation de l'association de l'ivabradine ou de la *N*-{[(7*S*)-3,4-diméthoxybicyclo[4.2.0]octa-1,3,5-trién-7-yl)méthyl}-3-(7,8-diméthoxy-1,2,4,5-tétrahydro-3*H*-3-benzazépin-3-yl)-*N*-méthyl-3-oxo-1-propanamine et du perindopril permet de ramener les paramètres physiologiques observés à des valeurs très proches de la normale. Ces observations permettent d'envisager l'utilisation de l'association de l'ivabradine ou de la *N*-{[(7*S*)-3,4-diméthoxybicyclo[4.2.0]octa-1,3,5-trién-7-yl]méthyl} -3-(7,8-diméthoxy-1,2,4,5-tétrahydro-3*H*-3-benzazépin-3-yl)-*N*-méthyl-3-oxo-1-propanamine et du perindopril dans le traitement de l'insuffisance cardiaque à fonction systolique conservée.

Le perindopril est utilisé tel quel ou sous forme d'un de ses sels d'addition à un acide ou à une base pharmaceutiquement acceptable, et plus particulièrement ses sels de *tert-*butylamine ou d'arginine, leurs hydrates et formes cristallines.

La présente invention s'étend également aux compositions pharmaceutiques contenant comme principes actifs :
- l'ivabradine ou l'un de ses hydrates, formes cristallines, et sels d'addition à un acide pharmaceutiquement acceptable et plus particulièrement son chlorhydrate, ou la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine ou l'un de ses sels d'addition à un acide pharmaceutiquement acceptable, et plus particulièrement son chlorhydrate et son fumarate, leurs hydrates ou formes cristallines, et
- le perindopril ou un de ses sels d'addition à une base pharmaceutiquement acceptable, et plus particulièrement ses sels de *tert*-butylamine ou d'arginine, leurs hydrates ou formes cristallines,
pour leur utilisation dans le traitement de l'insuffisance cardiaque à fonction systolique conservée.

Les compositions pharmaceutiques pouvant être utilisés sont celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc... ainsi que les compositions pharmaceutiques avec libération programmée, retardée, prolongée ou différée.

Outre l'ivabradine ou la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine et le perindopril, lesdites compositions pharmaceutiques contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer:
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne de 2,5 à 30 mg d'ivabradine par 24 heures et plus préférentiellement de 5 à 15 mg par jour et de manière encore préférée de 10 à 15 mg par jour. La dose de N-{[(7S)-3,4-diméthoxybicyclo[4.2.0]octa-1,3,5-trién-7-yl]méthyl}-3-(7,8-diméthoxy-1,2,4,5-tétrahydro -3H-3-benzazépin-3-yl)-N-méthyl-3-oxo-1-propanamine (ci après dénommé composé A) pourra varier de 5 à 100 mg par jour. La dose du perindopril pourra être moindre que celle utilisée lorsqu'il est administré seul.

La dose journalière de perindopril sera préférentiellement comprise entre 1 et 10 mg inclus.

Les exemples suivants illustrent l'invention.

### Liste des abréviations utilisées

- dP/dtₘₐₓ: : augmentation maximale de pression par seconde
- dP/dtₘᵢₙ: : diminution maximale de pression par seconde
- IC: : insuffisance cardiaque
- LVEDP: : Left Ventricular End Diastolic Pressure (pression télé-diastolique du ventricule gauche)
- LVEDPVR: : Left Ventricular End Diastolic Pressure Volume Relation (relation pression-volume télé-diastolique du ventricule gauche)
- LVESP: : Left Ventricular End Systolic Pressure (pression télé-systolique du ventricule gauche)
- LVESPVR: : Left Ventricular End Systolic Pressure Volume Relation (relation pression-volume télé-systolique du ventricule gauche)
- VG: : ventricule gauche

### Tests pharmacologiques :

Une insuffisance cardiaque est provoquée chez des rats en pratiquant une ligature de l'artère coronaire gauche (les animaux témoins subissent une opération mais ne sont pas ligaturés) qui provoque une ischémie d'une partie de la paroi du ventricule gauche. Les animaux récupèrent pendant 7 jours puis, pendant 12 semaines, reçoivent soit 3 mg/kg du composé A, soit 0.4 mg/kg de perindopril, soit concomitamment perindopril et le composé A.

Douze semaines après l'opération, on constate que les animaux ayant subi la ligature coronaire développent une insuffisance cardiaque à la fois systolique (anomalie de l'éjection) et diastolique (anomalie du remplissage).

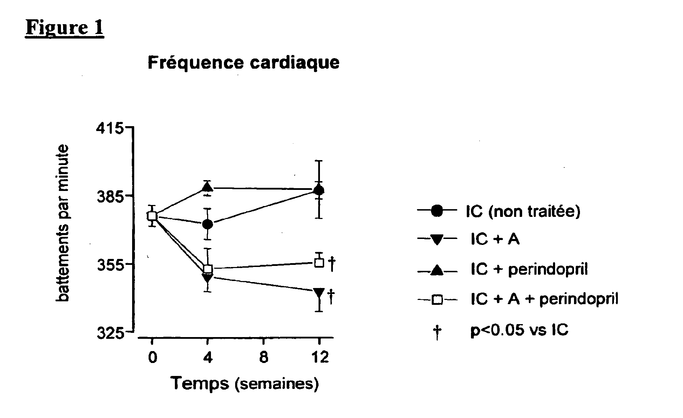

Chez ces animaux, le composé A permet de diminuer significativement la fréquence cardiaque, seul ou associé à perindopril (tableau 1 et figure 1).

**Tableau 1**

| | | | **IC (non traitée)** | **IC + A** | **IC + perindopril** | **IC + A + perindopril** |
|---|---|---|---|---|---|---|
| **Fréquence cardiaque** (bpm) | durée de traitement | 4 semaines | 372,5 | 349,3 | 388,2 | 352,8 |
| | | 12 semaines | 387,2 | 342,7^{†} | 387,7 | 353,1^{†} |

| | | | | | | |
|---|---|---|---|---|---|---|
| ^{†} p<0,05 vs IC | | | | | | |

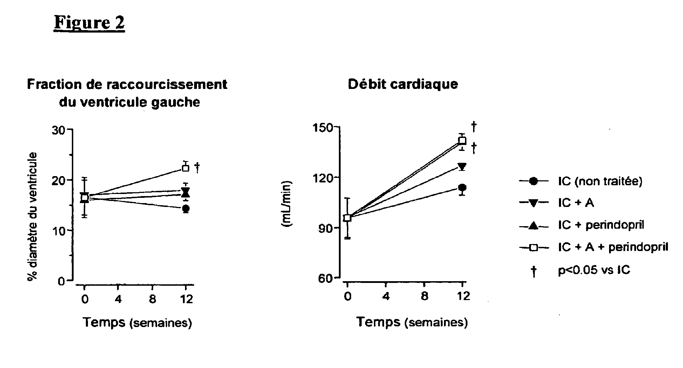

Le co-traitement par perindopril et le composé A permet de significativement augmenter la fraction de raccourcissement du ventricule gauche, c'est à dire d'améliorer sa contractilité (tableau 2 et figure 2). En conséquence, le débit cardiaque est amélioré par rapport aux animaux insuffisants cardiaques ne recevant pas de traitement.

**Tableau 2**

| | **IC (non traitée)** | **IC + A** | **IC + perindopril** | **IC + A + perindopril** |
|---|---|---|---|---|
| **fraction de raccourcissement** (% diamètre du VG) | 14,4 | 18,0 | 17,1 | 22,3^{†} |
| **débit cardiaque** (mL/min) | 114 | 127 | 142^{†} | 140^{†} |

| | | | | |
|---|---|---|---|---|
| ^{†} p<0,05 vs IC | | | | |

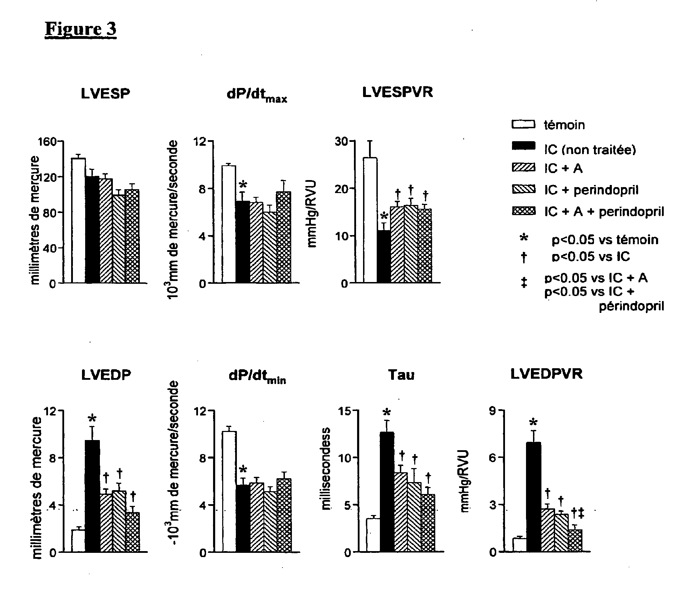

Comme le montre le tableau 3 (figure 3), les différents paramètres systoliques et diastoliques sont modifiés par l'insuffisance cardiaque. Le ventricule gauche se contracte moins bien (dP/dtₘₐₓ et LVESPVR significativement plus faibles chez les animaux IC que chez les témoins sains), ce qui dénote l'atteinte systolique. La fonction diastolique est très altérée: la pression à l'intérieur du ventricule en fin de diastole est élevée (LVEDP), le temps de relaxation (tau) est allongé et la compliance (capacité du ventricule à se distendre) est faible (LVEDPVR augmentée).

**Tableau 3**

| | **témoin** | **IC (non traitée)** | **IC + A** | **IC + perindopril** | **IC + A + perindopril** |
|---|---|---|---|---|---|
| **LVESP** (mm Hg) | 140 | 120 | 118 | 99 | 105 |
| **dP/dt**ₘₐₓ (10³mm Hg/s) | 9,92 | 6,89* | 6,78 | 5,97 | 7,69 |
| **LVESPVR** (mm Hg/RVU) | 26,4 | 11,1* | 16,1^{†} | 16,4^{†} | 15,6^{†} |
| **LVEDP** (mm Hg) | 1,86 | 9,43* | 4,89^{†} | 5,17^{†} | 3,32^{†} |
| **dP/d**tₘᵢₙ (-10³mm Hg/s) | 10,24 | 5,66* | 5,87 | 5,11 | 6,19 |
| **tau** (ms) | 3,54 | 12,64* | 8,37^{†} | 7,31^{†} | 6,05^{†} |
| **LVEDPVR** (mm Hg/RVU) | 0,84 | 6,93* | 2,70^{†} | 2,36^{†} | 1,37^{†‡} |

| | | | | | |
|---|---|---|---|---|---|
| * p<0,05 vs témoin; ^{†} p<0,05 vs IC; ^{‡} p<0,05 vs IC+A and vs IC+périndopril | | | | | |

On constate que le traitement des animaux insuffisants cardiaques, que ce soit par perindopril seul ou par le composé A seul, améliore la fonction systolique, ce que l'on peut observer avec la LVESPVR, seul paramètre indépendant des charges.

La pression télé-diastolique et le temps de relaxation sont nettement améliorés par perindopril seul ou par le composé A seul et on note une tendance à réduire encore ces deux paramètres lorsque les deux produits sont administrés ensemble. La compliance du ventricule gauche (mesurée par la LVEDPVR), seul paramètre indépendant de la charge, est très nettement améliorée par perindopril et par le composé A. Etonnamment, cet effet est significativement augmenté lorsque les animaux reçoivent concomitamment les deux traitements.

En effet, l'association du composé A et de perindopril permet d'améliorer significativement la compliance qui revient à un seuil proche de celui des animaux témoins.

L'association de perindopril et de la N-{[(7S)-3,4-diméthoxybicyclo[4.2.0]octa-1,3,5-trién-7-yl]méthyl}-3-(7,8-diméthoxy-1,2,4,5-tétrahydro-3H-3-benzazépin-3-yl)-N-méthyl-3-oxo-1-propanamine permet donc d'améliorer la fonction diastolique altérée.

Cet effet sur les fonctions systolique et diastolique a ensuite été étudié avec l'association de perindopril et d'un autre inhibiteur du courant I_{f}, l'ivabradine.

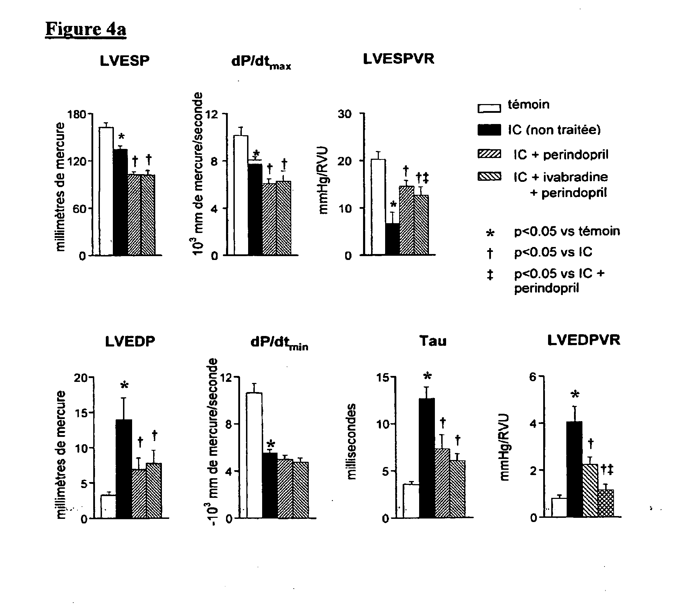

On constate que le traitement par perindopril seul ou associé à ivabradine améliore la fonction systolique (tableau 4a et figure 4a).

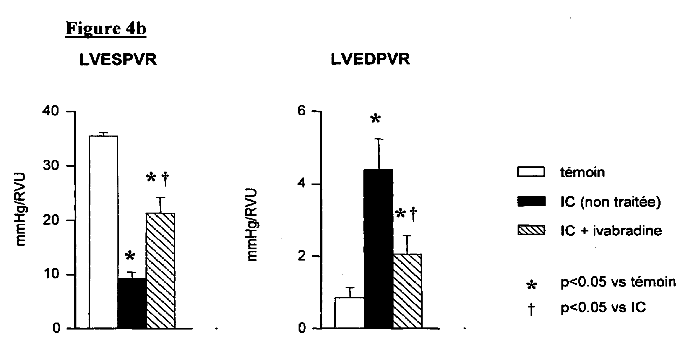

Le traitement par perindopril et ivabradine est nettement plus efficace que perindopril seul sur la dysfonction diastolique (l'effet de l'ivabradine seule est comparable à celui de perindopril seul, cf tableau 4b et figure 4b). La compliance du ventricule gauche est rétablie à un niveau similaire à celui des animaux sains.

**Tableau 4a**

| | **témoin** | **IC (non traitée)** | **IC + perindopril** | **IC + ivabradine + perindopril** |
|---|---|---|---|---|
| **LVESP** (mm Hg) | 163 | 134* | 102^{†} | 1.00^{†} |
| **dP/dt**ₘₐₓ (10³mm Hg/s) | 10,11 | 7,68* | 6,08^{†} | 6,10^{†} |
| **LVESPVR** (mm Hg/RVU) | 20,2 | 6,6* | 14,5^{†} | 12,6^{†‡} |
| **LVEDP** (mm Hg) | 3,29 | 13,93* | 6,88^{†} | 5,01^{†} |
| **dP/dt**ₘᵢₙ (-10³mm Hg/s) | 10,63 | 5,54* | 4,99 | 4,97 |
| **tau** (ms) | 3,21 | 14,29* | 10,92^{†} | 8,52^{†} |
| **LVEDPVR** (mm Hg/RVU) | 0,79 | 4,06* | 2,25^{†} | 1,15^{†‡} |

| | | | | |
|---|---|---|---|---|
| * p<0,05 vs témoin; ^{†} p<0,05 vs IC; ^{‡} p<0,05 vs IC+A et vs IC+périndopril | | | | |

**Tableau 4b**

| | **témoin** | **IC (non traitée)** | **IC + ivabradine** |
|---|---|---|---|
| **LVESPVR** (mm Hg/RVU) | 35,53 | 9,66* | 20.63*^{†} |
| **LVEDPVR** (mm Hg/RVU) | 0,85 | 5,33* | 1,87*^{†} |

| | | | |
|---|---|---|---|
| * p<0,05 vs témoin; ^{†} p<0,05 vs IC | | | |

Ces expériences montrent que, dans un modèle d'insuffisance cardiaque, l'association de l'ivabradine ou de la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine et du perindopril permet une amélioration de la fonction diastolique supérieure à celle obtenue avec l'un de ces deux traitements utilisé seul; cette amélioration permet de retrouver une fonction diastolique normale.

### Compositions pharmaceutiques :

### Formule de préparation pour 1000 comprimés dosés à 7,5 mg d'ivabradine et 2 mg de perindopril, tert-butylamine:

| | |
|---|---|
| Ivabradine, chlorhydrate | 8,085 g |
| Perindopril, *tert*-butylamine | 2 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

### Formule de préparation pour 1000 comprimés dosés à 10 mg de composé A et 2 mg de perindopril, tert-butylamine:

| | |
|---|---|
| Composé A, fumarate | 12,48 g |
| Perindopril, *tert*-butylamine | 2 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

D'autres exemples de compositions pharmaceutiques selon l'invention sont donnés ci-dessous, à titre non limitatif :

| **Exemple** | **Ivabradine (mg)** | **Composé A (mg)** | **Perindopril, sel de *tert-*butylamine (mg)** | **Perindopril, sel d'arginine (mg)** |
|---|---|---|---|---|
| 1 | 10 | - | 2 | - |
| 2 | 15 | - | 4 | - |
| 3 | 10 | - | - | 2,5 |
| 4 | 15 | - | - | 5 |
| 5 | - | 60 | 2 | - |
| 6 | - | 80 | 4 | - |
| 7 | - | 60 | - | 2,5 |
| 8 | | 80 | - | 5 |

## Revendications

1. Association de :
- l'ivabradine ou 3-{3-[{[(7S)-3,4-diméthoxybicyclo[4.2.0]octa-1,3,5-trién-7-yl] méthyl}(méthyl)amino]propyl}-7,8-di-méthoxy-1,3,4,5-tétrahydro-2*H*-3-benzazépin-2-one, ou l'un de ses sels d'addition à un acide pharmaceutiquement acceptable, leurs hydrates et formes cristallines, ou
- la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-méthyl-3-oxo-1-propanamine, ou l'un de ses sels d'addition à un acide pharmaceutiquement acceptable, leurs hydrates et formes cristallines.
avec le perindopril, ou l'un de ses sels d'addition à une base pharmaceutiquement acceptable, leurs hydrates ou formes cristallines,
pour son utilisation dans le traitement de l'insuffsance cardiaque à fonction systolique conservée.

2. Association selon la revendication 1 pour son utilisation dans une méthode de traitement selon la revendication 1, **caractérisée en ce que** l'ivabradine est sous la forme d'un chlorhydrate, ou de l'un de ses hydrates ou formes cristallines.

3. Association selon la revendication 1 pour son utilisation dans une méthode de traitement selon la revendication 1, **caractérisée en ce que** la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-methyl-3-oxo-1-propanamineest sous la forme d'un chlorhydrate ou d'un fumarate, ou de l'un de leurs hydrates ou formes cristallines.

4. Association selon l'une des revendications 1 à 3 pour son utilisation dans une méthode de traitement selon la revendication 1, **caractérisée en ce que** le perindopril est sous la forme d'un sel de *tert*-butylamine ou d'arginine ou de l'un de leurs hydrates ou formes cristallines.

5. Association selon la revendication 1 pour son utilisation dans une méthode de traitement selon la revendication 1, **caractérisée en ce que** l'ivabradine est sous forme de chlorhydrate ou de l'un de ses hydrates ou formes cristallines, et le perindopril est sous forme de sel de *tert*-butylamine ou d'arginine ou de l'un de leurs hydrates ou formes cristallines.

6. Association selon la revendication 1 pour son utilisation dans une méthode de traitement selon la revendication 1, **caractérisée en ce que** la *N*-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-methyl-3-oxo-1-propanamineest sous forme de chlorhydrate ou de fumarate ou de l'un de leurs hydrates ou formes cristallines, et le perindopril est sous forme de sel de *tert*-butylamine ou d'arginine ou de l'un de leurs hydrates ou formes cristallines.

7. Compositions pharmaceutiques contenant comme principes actifs :
- l'ivabradine, sous forme de chlorhydrate ou de l'un de ses hydrates ou formes cristallines, et
- le perindopril, sous forme de sel de *tert*-butylamine ou d'arginine ou de l'un de leurs hydrates ou formes cristallines,
seuls ou en combinaison avec un ou plusieurs excipients pharmaceutiquement acceptables,
pour leur utilisation dans le traitement de l'insuffisance cardiaque à fonction systolique conservée.

8. Compositions pharmaceutiques contenant comme principes actifs :
- la N-{[(7*S*)-3,4-diméthoxybicyclo[4.2.0]octa-1,3,5-trién-7-yl]méthyl}-3-(7,8-diméthoxy-1,2,4,5-tétrahydro-3*H*-3-benzazépin-3-yl)-*N*-methyl-3-oxo-1-propanamine, sous forme de chlorhydrate ou de fumarate ou de l'un de leurs hydrates ou formes cristallines, et
- le perindopril, sous forme de sel de *tert*-butylamine ou d'arginine ou de l'un de leurs hydrates ou formes cristallines,
seuls ou en combinaison avec un ou plusieurs excipients pharmaceutiquement acceptables, pour leur utilisation dans le traitement de l'insuffisance cardiaque à fonction systolique conservée.

## Patentansprüche

1. Kombination aus:
- Ivabradin oder 3-{3-[{[(7S)-3,4-Dimethoxybicyclo[4.2.0]octa-1,3,5-trien-7-yl]-methyl}(methyl)-amino]-propyl}-7,8-dimethoxy-1,3,4,5-tetrahydro-2*H*-3-benz-azepin-2-on oder eines seiner Additionssalze mit einer pharmazeutisch annehmbaren Säure, ihren Hydraten oder Kristallformen, oder
- N-{[(7*S*)-3,4-Dimethoxybicyclo[4.2.0]octa-1,3,5-trien-7-yl]-methyl}-3-(7,8-dimethoxy-1,2,4,5-tetrahydro-3H-3-benzazepin-3-yl)-*N*-methyl-3-oxo-1-propanamin oder eines seiner Additionssalze mit einer pharmazeutisch annehmbaren Säure, ihren Hydraten und Kristallformen,
und Perindopril oder eines seiner Additionssalze mit einer pharmazeutisch annehmbaren Base, ihren Hydraten oder Kristallformen,
zur Verwendung bei der Behandlung von Herzinsuffizienz mit beibehaltener systolischer Funktion.

2. Kombination nach Anspruch 1 zur Verwendung bei einer Methode zur Behandlung nach Anspruch 1, **dadurch gekennzeichnet, dass** Ivabradin in Form des Hydrochlorids oder eines seiner Hydrate oder Kristallformen vorliegt.

3. Kombination nach Anspruch 1 für die Verwendung bei einer Behandlungsmethode nach Anspruch 1, **dadurch gekennzeichnet, dass** *N*-{[(7*S*)-3,4-Dimethoxybicyclo[4.2.0]octa-1,3,5-trien-7-yl]-methyl}-3-(7,8-dimethoxy-1,2,4,5-tetrahydro-3*H*-3-benzazepin-3-yl)-*N*-methyl-3-oxo-1-propanamin in Form eines Hydrochlorids oder eines Fumarats oder eines ihrer Hydrate oder Kristallformen vorliegt.

4. Kombination nach einem der Ansprüche 1 bis 3 zur Verwendung bei einer Behandlungsmethode nach Anspruch 1, **dadurch gekennzeichnet, dass** Perindopril in Form eines *tert*.-Butylamin- oder Argininsalzes oder eines ihrer Hydrate oder Kristallformen vorliegt.

5. Kombination nach Anspruch 1 zur Verwendung bei einer Behandlungsmethode nach Anspruch 1, **dadurch gekennzeichnet, dass** Ivabradin in Form des Hydrochlorids oder eines seiner Hydrate oder Kristallformen und Perindopril in Form des *tert*.-Butylamin- oder Argininsalzes oder eines ihrer Hydrate oder Kristallformen vorliegen.

6. Kombination nach Anspruch 1 zur Verwendung bei einer Behandlungsmethode nach Anspruch 1, **dadurch gekennzeichnet, dass** *N*-{[(7*S*)-3,4-Dimethoxybicyclo[4.2.0]octa-1,3,5-trien-7-yl]-methyl}-3-(7,8-dimethoxy-1,2,4,5-tetrahydro-3*H*-3-benzazepin-3-yl)-*N*-methyl-3-oxo-1-propanamin in Form des Hydrochlorids oder des Fumarats oder eines ihrer Hydrate oder Kristallformen und Perindopril in Form des *tert*.-Butylamin- oder Argininsalzes oder eines ihrer Hydrate oder Kristallformen vorliegen.

7. Pharmazeutische Zubereitungen enthaltend als Wirkstoffe:
- Ivabradin in Form des Hydrochlorids oder eines seiner Hydrate oder Kristallformen, und
- Perindopril in Form des *tert*.-Butylamin- oder Argininsalzes oder eines ihrer Hydrate oder Kristallformen,
allein oder in Kombination mit einem oder mehreren pharmazeutisch annehmbaren Hilfsstoffen,
zur Verwendung bei der Behandlung von Herzinsuffizienz mit beibehaltener systolischer Funktion.

8. Pharmazeutische Zubereitungen enthaltend als Wirkstoffe:
- *N*-{[(7*S)*-3,4-Dimethoxybicyclo[4.2.0]octa-1,3,5-trien-7-yl]-methyl}-3-(7,8-dimethoxy-1,2,4,5-tetrahydro-3*H*-3-benzazepin-3-yl)-*N*-methyl-3-oxo-1-propanamin in Form des Hydrochlorids oder Fumarats oder eines ihrer Hydrate oder Kristallformen, und
- Perindopril in Form des *tert*.-Butylamin- oder Argininsalzes oder eines ihrer Hydrate oder Kristallformen,
allein oder in Kombination mit einem oder mehreren pharmazeutisch annehmbaren Hilfsstoffen zur Verwendung bei der Behandlung von Herzinsuffizienz mit beibehaltener systolischer Funktion.

## Claims

1. Association of:
- ivabradine, or 3-{3-[{[(7*S*)-3,4-dimethoxybicyclo[4.2.0]octa-1,3,5-trien-7-yl]-methyl}(methyl)amino]propyl}-7,8-dimethoxy-1,3,4,5-tetrahydro-2*H*-3-benzazepin-2-one, or one of its addition salts with a pharmaceutically acceptable acid, their hydrates and crystalline forms, or
- *N*-{[(7*S*)-3,4-dimethoxybicyclo[4.2.0]octa-1,3,5-trien-7-yl]methyl}-3-(7,8-dimethoxy-1,2,4,5-tetrahydro-3*H*-3-benzazepin-3-yl)-*N*-methyl-3-oxo-1-propanamine, or one of its addition salts with a pharmaceutically acceptable acid, their hydrates and crystalline forms,
with perindopril, or one of its addition salts with a pharmaceutically acceptable base, their hydrates or crystalline forms,
for use in the treatment of heart failure with preserved systolic function.

2. Association according to claim 1 for use in a method of treatment according to claim 1, **characterised in that** the ivabradine is in the form of a hydrochloride, or one of its hydrates or crystalline forms.

3. Association according to claim 1 for use in a method of treatment according to claim 1, **characterised in that** the *N*-{[(7*S*)-3,4-dimethoxybicyclo[4.2.0]octa-1,3,5-trien-7-yl]methyl}-3-(7,8-dimethoxy-1,2,4,5-tetrahydro-3*H*-3-benzazepin-3-yl)-*N*-methyl-3-oxo-1-propanamine is in the form of a hydrochloride or fumarate, or one of their hydrates or crystalline forms.

4. Association according to one of claims 1 to 3 for use in a method of treatment according to claim 1, **characterised in that** the perindopril is in the form of a *tert-*butylamine or arginine salt or one of their hydrates or crystalline forms.

5. Association according to claim 1 for use in a method of treatment according to claim 1, **characterised in that** the ivabradine is in the form of the hydrochloride or one of its hydrates or crystalline forms, and the perindopril is in the form of the *tert*-butylamine or arginine salt or one of their hydrates or crystalline forms.

6. Association according to claim 1 for use in a method of treatment according to claim 1, **characterised in that** the *N*-{[(7*S*)-3,4-dimethoxybicyclo[4.2.0]octa-1,3,5-trien-7-yl]methyl}-3-(7,8-dimethoxy-1,2,4,5-tetrahydro-3*H*-3-benzazepin-3-yl)-*N*-methyl-3-oxo-1-propanamine is in the form of the hydrochloride or fumarate or one of their hydrates or crystalline forms, and the perindopril is in the form of the *tert*-butylamine or arginine salt or one of their hydrates or crystalline forms.

7. Pharmaceutical compositions comprising as active ingredients:
- ivabradine, in the form of the hydrochloride or one of its hydrates or crystalline forms, and
- perindopril, in the form of the *tert*-butylamine or arginine salt or one of their hydrates or crystalline forms,
on their own or in combination with one or more pharmaceutically acceptable excipients,
for use in the treatment of heart failure with preserved systolic function.

8. Pharmaceutical compositions comprising as active ingredients:
- *N*-{[(7*S*)-3,4-dimethoxybicyclo[4.2.0]octa-1,3,5-trien-7-yl]methyl}-3-(7,8-dimethoxy-1,2,4,5-tetrahydro-3*H*-3-benzazepin-3-yl)-*N*-methyl-3-oxo-1-propanamine, in the form of the hydrochloride or fumarate or one of their hydrates or crystalline forms, and
- perindopril, in the form of the *tert*-butylamine or arginine salt or one of their hydrates or crystalline forms,
on their own or in combination with one or more pharmaceutically acceptable excipients, for use in the treatment of heart failure with preserved systolic function.
